# EUROPEAN PATENT SPECIFICATION

(11) **EP 1 032 364 B1**
(45) Date of publication and mention of the grant of the patent: **03.03.2004**
(21) Application number: 99937489.5
(22) Date of filing: 26.07.1999
(51) Int. Cl.: A61K 7/48, C07H 3/06

(54) **TOPICAL COMPOSITIONS CONTAINING SIALYL SUGARS AND THEIR DERIVATIVES**
SIALYLZUCKER UND IHRE DERIVATEN ENTHALTENDE TOPISCHE ZUBEREITUNGEN
COMPOSITIONS TOPIQUES CONTENANT DES SUCRES SIALYLES ET LEURS DERIVES

(30) Priority: 27.07.1998 US 123251
(43) Date of publication of application: 06.09.2000
(73) Proprietor: E-L Management Corp., New York, NY 10153 (US); Neose Technologies, Inc., Horsham, PA 19044 (US)
(72) Inventor: Anderson, Jon, Galesbury, Michigan49053 (US); DeFrees, Shawn, Horsham, PA 19044 (US)
(74) Representative: Horner, Martin Grenville
(86) International application number: PCT/US1999/016884
(87) International publication number: WO 2000/006115

(56) References cited:
- WO-A-93/24505
- WO-A-96/19231
- WO-A-98/48817
- DATABASE CHEMICAL ABSTRACTS [Online] STN abstract 111: 239 563, XP002121522 cited in the application & JP 01 163125 A (SHISEIDO CO., LTD)
- DATABASE CHEMICAL ABSTRACTS [Online] stn abstract 114:49368, XP002121523 & JP 02 229103 A (T. HORIUCHI ET AL.)

## Description

### 1. FIELD OF THE INVENTION

The invention relates to the use of sialyl lactose for regulating inflammatory responses and treating skin irritation.

### 2. BACKGROUND OF THE INVENTION

### 2.1. SKIN IRRITATION AND ASSOCIATED INFLAMMATORY RESPONSES

Skin irritation can have any of several causes, for example, skin irritation can result from diseases, such as psoriasis, eczema, and seborrheic dermatitis. Psoriasis is a chronic skin condition that is characterized by red plaques with thick, flaking, silvery scales. Affected areas can range from localized patches of skin to the entire body. Psoriasis is not presently curable and even temporary relief requires long-term treatment. Eczema and seborrheic dermatitis are also inflammatory conditions of the skin, and typically involve scaling or blisters. Eczema may affect up to 10% of the adult population and as many as 20% of children at any given time.

Exposure to an irritant or allergen can also result in skin irritation and inflammation. These allergic conditions are often referred to as contact dermatitis and can be caused by, for example, exposure to substances found in cosmetics, clothing, plants and household items. Administration of certain drugs and by particular means of administration, e.g., transdermal delivery, can also cause irritation. Atopic dermatitis is typically associated with a family history of allergic rhinitis, asthma, or atopic eczema.

Skin irritation can also result from exposure to certain plants. For example, exposure to oils from poison ivy, poison oak, the poison sumac can result in redness and swelling, followed by blisters and severe itching, in persons who are sensitive to these plants. Another cause of skin irritation is insect bites and stings, which can cause reactions such as hives, inflammatory papulae, and blisters.

Inflammatory responses, including those that are associated with many forms of skin irritation, are usually caused by activation of the immune system. Recent research indicates that specific cell surface oligosaccharide structures are involved in the activation and regulation of immune responses. Specific sialylated oligosaccharides function as ligands for intercellular adhesion receptors, matrix proteins and growth factors. For example, compounds bearing the oligosaccharide moiety sialyl-LewisX(SLeX; NeuAcα2-3Galβ1-4(Fucα 1-3) GlcNAcβ1 were recently found to play an important role in the inflammatory response, mediating the cell adhesion of leukocytes to endothelial cells (see, e.g., US Patent No. 5,753,631). After binding to endothelial cells, the leukocytes are able to squeeze past gaps between them to enter the adjoining tissue, where they help repair injury.

Sialylated oligosaccharide structures are also involved in macrophage-mediated immune responses. Macrophage cell surfaces display sialoadhesin, which is a 185 kDa transmembrane glycoprotein that mediates cell-cell interactions through recognition of glycoproteins and glycolipids that contain the terminal oligosaccharide NeuAcα2-3GalNac (van den Berg *et al.*, 1992, *J. Exp. Med.* 176: 647-755; Crocker *et al*., 1991, *EMBO.J* 10; 1661-1669). Sialoadhesin, which binds preferentially to granulocytic cells (Crocker *et al*., 1995, *J. Clin. Invest.* 95: 635-643), is a member of the immunoglobulin superfamily (Crocker *et al*., 1994, *EMBO J*. 13: 4490-4503). The amino acid sequence of sialoadhesion is similar to those of CD22 and the myelin-associated glycoprotein (MAG), both of which also bond sialylated oligosaccharides (Kelm *et al*., 1994, *Curr. Biol.* 4; 965-972). These three molecules, which have been termed the "sialoadhesin family" of the immunoglobulin superfamily (Id.), are selective in the cell types that are bound. This selectivity is due at least in part to the preference of each member of the sialoadhesin family for a specific oligosaccharide structure. While sialoadhesin preferentially binds N- and O-linked oligosaccharides that terminate in α2, 3-linked sialic acid, MAG preferentially binds O-linked α2, 3-sialylated oligosaccharides and β22 binds to N-linked α2, 6-sialylated oligosaccharides (*Id*.) For a review of the role of sialoadhesin in macrophage recognition, *see e.g*., Crocker *et al*., 1997, *Glycoconjugate J*. 14: 601-609).

### 2.2. OLIGOSACCHARIDES AND THEIR ROLE IN CELL ADHESION

Cell surface oligosaccharides have recently been found to have important regulatory roles in cell biological and immunologic processes. Specific oligosaccharides have been shown to function as ligands for intercellular adhesion receptors, matrix proteins and growth factors. In particular, compounds bearing the oligosaccharide moiety, sialyl-Lewis X (SLeX), were recently found to play an important role in the inflammatory response, mediating the cell adhesion of leukocytes to endothelial cells.

Leukocytes migrate into tissues during an inflammatory response. Vascular endothelial cells' participation in the recruitment of leukocytes to injured tissue occurs by the expression of cell adhesion molecules in response to cytokines (Pober et al., 1986, *J. Immunol.* 136:1680; Bevilacqua et al., 1987, *Proc. Natl. Acad. Sci.* 84:9236). The cell adhesion molecules make the endothelial cells recognizable to SLeX containing white blood cells. After binding to endothelial cells, the leukocytes are able to squeeze past gaps between them to enter the adjoining tissue, where they help repair injury. Sometimes too many leukocytes are recruited and normal tissue is destroyed. This can occur in septic shock, in chronic inflammatory diseases such as psoriasis and rheumatoid arthritis, and in reperfusion tissue injury that occurs following heart attack, stroke or organ transplant, (Malech et al., 1987, *N. Engl. J. Med.* 317:687; Cotran et al., 1986, *J. Exp. Med.* 164:661).

The biological mechanism of leukocytes binding to activated endothelial cells involves binding by SLeX to the endothelial leukocyte adhesion molecule-1 (ELAM-1), the cell adhesion molecule synthesized and expressed on endothelial cell surfaces in response to cytokines, interleukin-1 and tumor necrosis factor (Phillips et al., 1990, *Science* 250:1130). ELAM-1 is produced within 2 to 4 hours of cytokine induction and mediates binding of neutrophils at endothelial foci (Bevilacqua et al., 1987, *Proc. Natl. Acad. Sci. U.S.A.* 84:9238).

ELAM-1 (E-selectin) is a member of the selectin family of cell adhesion molecules including Mel-14/LAM-1 (L-selectin) and GMP-140/PADGEM (P-selectin), which also mediate cell-cell interactions (Bevilacqua et al., 1989, *Science* 243:1160; Lasky et al., 1989, *Cell* 56:1045). Because the NH₂ terminal domains of all three selections have a lectin motif they were postulated to recognize a carbohydrate motif. The SLeX group has been found to serve as a common ligand for P- and E-selections and is a component of glycosphingolipids and glycoproteins which are also found on the surface of the leukocyte membrane. The ligand for L-selectin has also been proposed to contain an SLeX type structure in which the sialic acid is replaced with a sulfate group. (Yuen et al., 1992, *Biochemistry* 31:7549).

The increased understanding of the role of carbohydrates as cell surface recognition elements, particularly in the immune response, has led to increased interest in the production of carbohydrate molecules of defined structure in order to regulate those interactions upon systemic administration. The use of SLeX carbohydrate groups has been proposed to block cell-cell adhesion for the treatment of acute and chronic inflammation (Paulson et al., 1990, *Science* 250:1130-1132). SLeX compounds have also been shown to inhibit binding to E-selectin (DeFrees et al., 1993, *J. Am. Chem. Soc*. 115:7549).

### 2.3. SIALYL OLIGOSACCHARIDES WITH IMMUNOMODULATING ACTIVITY

Much of the focus in the art has been directed to developing large SLeX analogs with immunomodulating properties for use as systemic anti-inflammatories. International Publication No. WO 92/22564, published December 12, 1992, discloses sulfate, phosphate and carboxylate derivatives of Lewis X oligosaccharides that lack a sialyl group, but are said to provide enhanced immunosuppressing or tolerogenic properties over derivatives lacking the sulfate, phosphate or carboxylate substituents. These compounds have utility in suppressing a cell-mediated immune inflammatory response to an antigen, and therefore reduce sensitization of a mammal to an immune response. Other groups have focused on inhibiting specific cellular interactions. In particular, Nashed et al. (see U.S. Patent No. 5,326,752, issued July 5, 1994) has developed substituted lactose and lactosamines that specifically bind to the ELAM-1 receptor and thus modulate inflammatory responses. Pharmaceutical compositions containing these compounds, are reported to have utility in the treatment of inflammation, allergic reactions and autoimmune disease upon parenteral administration.

In particular, International Publication No. WO91/19501, published December 26, 1991, and International Publication No. WO91/19502, published December 26, 1991, disclose that oligosaccharides containing the pentameric and hexameric structures shown below inhibited the selective intracellular binding between cells containing the ligand (below) and those containing a selectin receptor and that the penta- and hexasaccharides provided better inhibition than did SLeX:
NeuAcα2-3βGalβ1-4(Fucα1-3)GlcNAcβ1,3Galβ-;
NeuAcα2-3βGalβ1-4(Fucα1-3)GlcNAcβ1-3Galβ1-4Glc-; and
NeuAcα2-3βGalβ1-4(Fucα1-3)GlcNAc = SLeX.
These oligosaccharides have utility in inhibiting selectin-mediated cell adhesion related to inflammatory disease and tumor metastasis, when administered intravenously, orally or as an aerosol.

Other research groups have extended the area of interest to compositions containing sialyl oligosaccharides derivative for use as systemic anti-inflammatories and cosmetics. For example, two Japanese patent applications, Yoshihiro et al. JP163110A, published June 27, 1989, and Yoshihiro et al., JP163125A, published June 27, 1989, describe the use of sialyl oligosaccharide derivative compounds in both systemic and topical applications. In both of these applications the sialic acid compounds are crudely extracted from milk preparations, resulting in complex mixtures of sialyl sugars with other sugars, proteins and fats. The isolation procedures described in the patent publications and the basis of the identification of sialic acid as part of the "sialic acid containing complexes" is based solely on the allegation that the isolated complex mixtures contained charged oligosaccharide functionalities. The patent publications describe the use of these crude preparations in a topical composition which allegedly has activity as a skin aging preventive medication and as a systemic anti-inflammation medication.

In addition, large oligosaccharides containing two copies of the SLeX group have been designed for use as anti-inflammatory agents. International Publication No. WO 95/03059, published February 2, 1995, discloses the use of bivalent SLeX compounds to inhibit cellular binding to a selectin receptor. These large oligosaccharides are used in compositions to inhibit adhesion between cells that express a selectin receptor such as the E selectin receptor and cells that express SLex on their surfaces.

Synthetic SLeX derivatives which are modified at the C-2 and/or C-6 position of the N-acetylglucosamine (GlcNAc) residue with immunomodulating properties have also been disclosed in the art. International Publication No. WO 92/22565, published December 23, 1992, describes methods for the synthesis of SLeX derivatives with such modifications made to the galactose and/or GlcNAc units of SLeX, employing specified sialyl and fucosyl transferases so that these derivatives are readily prepared by sequential enzymatic methods *in vitro*. These SLeX derivatives have use in compositions to systemically treat and prevent disease by modulating the immune response.

The development of topical compositions containing SLeX derivatives previously has attracted little interest which is probably due to the size of the molecule and the cost and difficulty in synthesizing, purifying and formulating such molecules into compositions suitable for topical application.

In view of the above, it would be particularly beneficial to develop commercially feasible compounds which possess anti-inflammatory properties, while being effective upon topical applications.

### 3. SUMMARY OF THE INVENTION

The present invention provides the use of sialyl lactose in regulating inflammatory responses, reducing skin irritation, and treating skin reddening.

The invention provides carbohydrate compounds that are capable of treating and preventing undesirable inflammatory responses and skin irritation. The compounds can be used in pharmaceutical compositions and methods of treatment.

The anti-inflammatory compounds are generally sialic acids or glycosides of sialic acids. The sialyl glycosides typically have a formula:
sialic acid-Z-R-P, wherein:
   Z is a member selected from the group consisting of alpha- or beta- linked O, S, and N-A, wherein A is a member selected from the group consisting of H, Acetyl ("Ac"), alkylacyl, aryl and acyl;
   R is a member selected from the group consisting of a carbohydrate, an aglycon, a polysaccharide, a polymer, an oligosaccharide, a lipid, a ceramide, and a sphingosine; and
   P is H or a member selected from the group consisting of a polysaccharide, a polymer, and an oligosaccharide.

These sialylated structures include both monovalent and polyvalent forms as shown in Compounds I and II below: 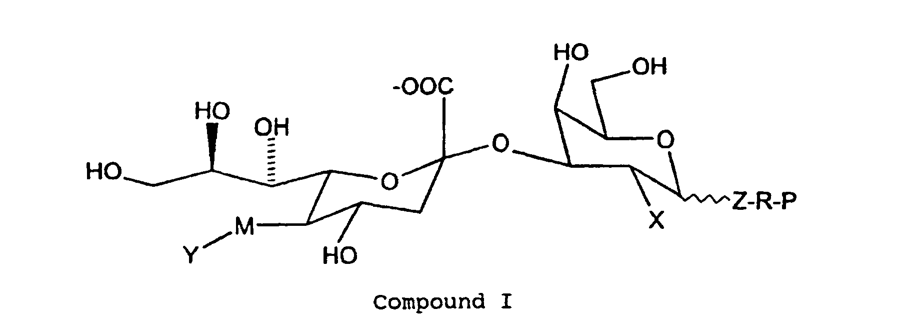 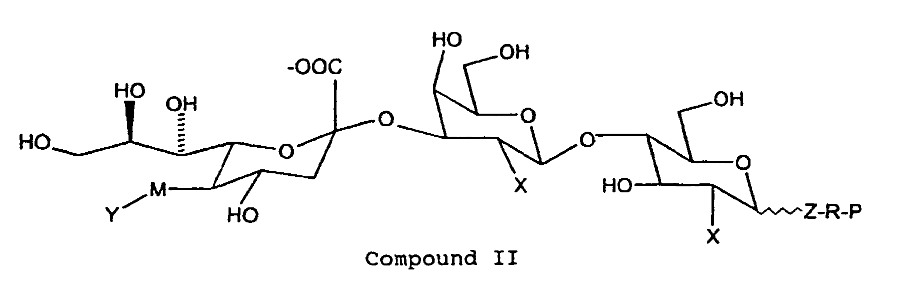 wherein Z, R and Ṗ are set forth above, and X is a member selected from the group consisting of OH, NH₂, NHAc, and N-A, wherein A is a member selected from the group consisting of H, Ac, alkylacyl, aryl and acyl; and
Y is a member selected form the group consisting of H, Ac, alkyl, aryl, alkylaryl, alkylacyl, arylalkylacyl, glycoyl, and an amino acid; and
M is a member selected from the group consisting of NH or O.

In Compound II, each X can be selected independently.

The invention encompasses the topical use lactose of sialyl 3'-sialyl lactose, 6'-sialyl lactose.

The present invention may be understood more fully by reference to the detailed description and illustrative examples which are intended to exemplify non-limiting embodiments of the invention.

### 4. DETAILED DESCRIPTION OF THE INVENTION

### 4.1. DEFINITIONS

As used herein "inflammation" refers to a reaction of either the specific and/or non-specific defense systems. A specific defense system reaction is a specific immune system reaction to an antigen. Exemplary of specific defense system reactions include antibody response to antigens, such as viruses, and delayed-type hypersensitivity. A non-specific defense system reaction is an inflammatory response mediated by leukocytes generally incapable of immunologic memory. Such cells include macrophages, eosinophils, and neutrophils. Examples of non-specific reactions include the immediate swelling after a bee sting, and the collection of PMN leukocytes at sites of bacterial infection.

As used herein, "oligosaccharide" refers to a carbohydrate structure having from 2 to about 10 saccharide units. The particular saccharide units employed are not critical and include, by way of example, all natural and synthetic derivatives of glucose, galactose, N-acetylglucosamine, N-acetylgalactosamine, fucose, sialic acid, and the like.

As used herein, "sialic acid" or "sialyl" refers to all naturally occurring structures of sialic acid and analogs of sialic acid including those wherein the sialic acid unit has been chemically modified so as to introduce and/or remove one or more functionalities. For example, such modifications can result in the removal of an -OH functionality, the introduction of an amine functionality, the introduction of a carboxylic acid or ester functionality, the introduction of a halo functionality, and the like. The term "sialic acid" refers to any member of a family of nine-carbon carboxylated sugars. The most common member of the sialic acid family is N-acetyl-neuraminic acid (2-keto-5-acetamindo-3-5-dideoxy-D-glycero-D-galactononulopyranos-1-onic acid (often abbreviated as Neu5Ac, NeuAc or NANA). A second member of the family is N-glycolylneuraminic acid (Neu5Gc or NeuGc), in which the N-acetyl group of NeuAc is hydroxylated. A third sialic acid family member is 2-keto-3-deoxy-nonulosonic acid (KDN) (Nadano *et al*. (1986) *J. Biol. Chem.* 261: 11550-11557; Kanamori et al., (1990) J. Biol. Chem. 265: 21811-21819. Also included are 9-substituted sialic acids such as a 9-O-C 1-C6 acyl-Neu5Ac like 9-0-lactyl- Neu5Ac or 9-0-acetyl-NeuSAc, 9-deoxy-9-fluoro-Neu5Ac and 9-deoxy-Neu5Ac. For review of the sialic acid family, *see*, *e.g*., Varki (1992) *Glycobiology* 2: 25-40; *Sialic Acids: Chemistry, Metabolism and Function,* R. Schauer, Ed. (Springer-Verlag, New York (1992). The synthesis and use of sialic acid compounds in a sialylation procedure is disclosed in international application WO 92/16640, published October 1, 1992, incorporated herein by reference in its entirety.

As used herein, "safe and effective amount" means an amount of the oligosaccharide, compound or composition, sufficient to significantly induce a positive modification in the condition to be treated. The safe and effective amount of the compound or composition may vary with the particular condition being treated, the age and physical condition of the patient being treated, the severity of the condition, the nature of concurrent treatment, the specific compound, compounds or composition employed, the particular pharmaceutically-acceptable carrier utilized, and like factors within the knowledge, and expertise of the attending physician or health care provider.

As used herein, "substantially pure" means that an object species, e.g., a sialyl glycoside, is the predominant species present and is essentially free of other components with which it is associated in its natural state. Preferably, a substantially purified fraction is a composition wherein the object species comprises at least about 50 percent (on a molar basis) of all macromolecular species present. More preferably, a substantially pure species is at least 90 to 95%, or is purified to essential homogeneity wherein the composition consists essentially of a single carbohydrate or macromolecular species.

As used herein, "cosmetic" means a formulation to be applied to the skin which improves the texture or appearance thereof, without necessarily affecting the structure or function of the skin, or rendering a benefit or an effect of treating or preventing an abnormal biological condition or a disease. A safe and effective amount of a cosmetic means an amount of the cosmetic sufficient to render a beneficial effect to the skin.

As used herein, "pharmaceutical" means a prophylactic or therapeutic formulation to be applied to the skin which renders a benefit or a therapeutic effect of treating or preventing an abnormal biological condition or disease.

### 4.2. SIALYL GLYCOSIDES

The present invention provides the use of sialyl lactose with anti-inflammatory properties and utility in pharmaceutical and cosmetic applications, especially topical applications.

The invention provides carbohydrate compounds that are capable of treating and preventing undesirable inflammatory responses and skin irritation. The compounds can be used in pharmaceutical compositions and methods of treatment.

The anti-inflammatory compounds are generally sialic acids or glycosides of sialic acids. The sialyl glycosides typically have a formula:
sialic acid-Z-R-P, wherein:
   Z is a member selected from the group consisting of alpha- or beta-linked O, S, and N-A, wherein A is a member selected from the group consisting of H, Ac, alkyl acyl, aryl and acyl;
   R is a member selected from the group consisting of a carbohydrate, a polysaccharide, a polymer, an oligosaccharide, a lipid, a ceramide, and a sphingosine; and
   P is H or a member selected from the group consisting of a polysaccharide, a polymer, and an oligosaccharide.

These sialylated structures include both monovalent and polyvalent forms as shown as Compounds I and II below: 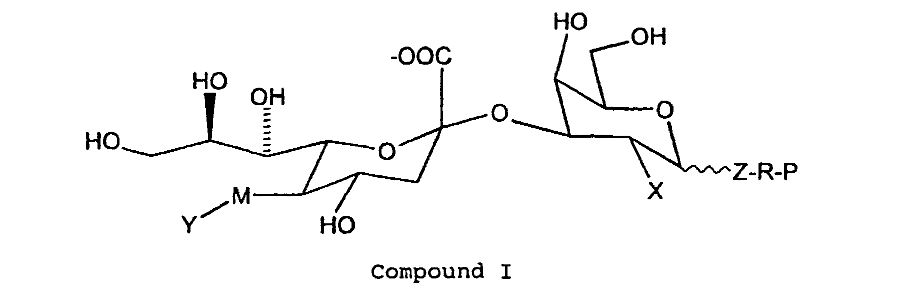 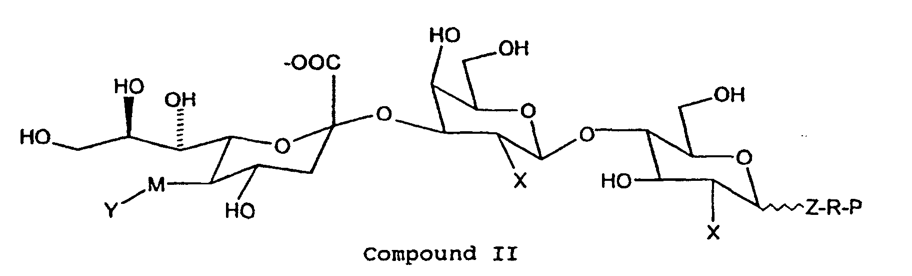 wherein Z, R and P are set forth above, and X is a member selected from the group consisting of OH, NH₂, NHAc, and N-A, wherein A is a member selected from the group consisting of H, Ac, alkylacyl, aryl and acyl; and
Y is a member selected form the group consisting of H, Ac, alkyl, aryl, alkylaryl, alkylacyl, arylalkylacyl, glycoyl, and an amino acid; and
M is a member selected from the group consisting of NH or O.

In Compound II, each X can be selected independently.

In the above descriptions, the terms are generally used according to their standard meanings. The term "alkyl" as used herein means a branched or unbranched, saturated or unsaturated, monovalent or divalent, hydrocarbon radical having from 1 to 20 carbons, including lower alkyls of 1-8 carbons such as methyl, ethyl, n-propyl, butyl, n-hexyl, and the like, cycloalkyls (3-7 carbons), cycloalkylmethyls (4-8 carbons). and arylalkyls. The term "alkoxy" refers to alkyl radicals attached to the remainder of the molecule by an oxygen, *e.g*., ethoxy, methoxy, or n-propoxy. The term "alkylthio" refers to alkyl radicals attached to the remainder of the molecule by a sulfur.

The term "acyl" refers to a radical derived from an organic acid by the removal of the hydroxyl group. Examples include acetyl, propionyl, oleoyl, myristoyl.

The term "aryl" refers to an aromatic monovalent carbocyclic radical having a single ring (e.g., phenyl) or two condensed rings (e.g., naphthyl), which can optionally be mono-, di-, or tri-substituted, independently, with alkyl, lower-alkyo, cycloalkyl, hydroxylower-alkyl, aminolower-alkyl, hydroxyl, thiol, amino, halo, nitro, lower-alkyulthio, lower-alkoxy, mono-lower-alkylamino, di-lower-alkylamino, acyl, hydroxycarbonyl, lower-alkoxycarbonyl, hydroxysulfonyl, lower-alkoxysulfonyl, lower-alkylsuylfonyl, lower-alkyulsulfinyl, triffluoromethyl, cyano, tetrazoyl, carbamoyl, lower-alklcarbamoyl, and di-lower-alkylcarbamoyl. Alternatively, two adjacent positions of the aromatic ring may be substituted with a methylenedioxy or ithylenedioxy group.

The invention encompasses sialyl lactose derivatives, including, benzyl sialyl lactose and ethyl sialyl lactose and pharmaceutically or cosmetically acceptable salts thereof.

All of the sialyl lactose of the present invention have utility in topical applications, both pharmaceutical and cosmetic compositions, in the treatment of disorders associated with the inflammatory response of cells in the skin and skin irritation.

### 4.3. SYNTHESIS

The compounds of the present invention can be synthesized in a number of ways, including in accordance with standard organic chemical techniques using readily/commercially available starting materials. Alternatively, the sialyl lactose of the present invention can be prepared from semisynthetic methods or completely from enzymatic synthesis. The sialyl lactose of the present invention may also be synthesized by a number of glycosyl transferase cycles, for example utilizing sialyl transferase, as described in U.S. Patent Nos. 5,374,541, 5,728,554, 5,753,631, EPO Publication No. 319,253, WO96/32492 and Paulson, et al., 1992, J. Am. Chem. Soc. 114:9283, each of which are incorporated herein by reference in their entirety.

In a preferred method (Scheme I below), a compound containing one or more carbohydrate acceptor units (Z-R-P in Scheme I) can be reacted sequentially with a galactosyltransferase (*e.g.*, N-acetylglucosamine β1-4 galactosyltransferase (E.C. 2.4.1.90)), a sialytransferase (Galβ1-4GlcNAcα2-3 sialytransferase (E.C. 2.4.99.6) or Galβ1-3GalNAca2-3 sialytransferase (E.C. 2.4.99.4) and, optionally, a fucosyltransferase (*e.g*., N-acetylglucosaminide α1-3 fucosyltransferase (E.C. 2.4.1.152)) to yield the desired sialyl galactoside structures. In this case, R can be a carrier moiety or activatable intermediate that will allow attachment to a suitable carrier moiety. Each enzymatic reaction uses the appropriate nucleotide sugar as a donor substrate to generate the following intermediates in the synthesis of the sialyl galactoside. The glycosyl transfer reactions may optimally be carried out with added alkaline phosphatase (e.g., from calf intestine, CIAP) to consume the nucleotide phosphate byproduct which may inhibit the reaction.

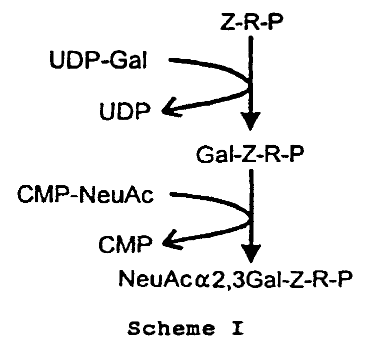

As shown in Scheme I, the steps for these methods typically include:
(a) galactosylating a suitable acceptor compound with a galactosyltransferase in the presence of a UDP-galactose under the conditions sufficient to form the compound:
   Gal-Z-R-P; and
(b) sialylating the compound formed in (a) with a sialyltransferase in the presence of a CMP derivative of a sialic acid using an α(2-3) or an α(2-6) sialyltransferase under conditions in which sialic acid is transferred to the non-reducing sugar to form the compound:
   NeuAcα(2-3)Gal-Z-R-P or NeuAcα(2-6)Gal-Z-R-P.

In these formulas, Z is a member selected from the group consisting of alpha- or beta- linked O, S, and N-A, wherein A is a member selected from the group consisting of H, Acetyl ("Ac"), alkylacyl, aryl and acyl;
R is a member selected from the group consisting of a carbohydrate, an aglycon, a polysaccharide, a polymer, an oligosaccharide, a lipid, a ceramide, and a sphingosine; and
P is H or a member selected from the group consisting of a polysaccharide, a polymer, and an oligosaccharide.

The general conditions for preparative enzymatic synthesis of sialyl galactosides are known (see, e.g., Toone *et al.*, 1989, *Tetrahedron* 45:5365-5422; Wong *et al*., 1982, *J. Am. Chem*. *Soc.* 47:5416-5418; Unverzagt *et al.*, 1990, *J. Am. Chem. Soc.* 112:9308-9309; Prieels *et al.,* 1981, *J. Biol*. *Chem*. 256:10456-10463. Each of the key enzymatic reactions has been demonstrated (Beyer *et al*., 1981, *Adv. Enzymol.* 52:23; Toone *et al*., *supra;* and Howard *et al.,* 1981, *J. Biol. Chem*. 262:16830-16837). For preparative reactions, the galactosyltransferase and the sialytransferase(s) are purified from natural sources (Beyer *et al., supra*, and Weinstein *et al*., 1982, *J. Biol. Chem.* 257:13835:13844). Fucosyltransferases may also be identified from natural sources, as generally described in Crawley and Hindsgaul, 1989, *Carbohyde. Res*. 193:249-256). The cDNAs of the galactosyltransferase and a sialytransferase have been cloned (Paulson and Colley, 1989, *J. Biol. Chem*. 264:17615-17618), allowing the production of soluble recombinant enzymes for large-scale preparative synthesis (Colley *et al*., 1989, *J. Biol. Chem*. 264:17619-17622).

### 4.4 METHODS FOR TREATING DISORDERS RELATED TO ANTI-INFLAMMATORY RESPONSES

The present invention also relates to a method for treating disorders treated by anti-inflammatory responses and skin inflammatory responses, including but not limited to those described herein. Such a method comprises applying to the skin a safe and effective amount of one or more of the sialyl lactose of the present invention.

A preferred method of treating skin disorders related to anti-inflammatory responses is via chronic topical application of a safe and effective amount of the sialyl oligosaccharide derivative to regulate the anti-inflammatory response. The amount of a composition containing the sialyl lactose of the present invention and frequency of topical application to the skin can vary widely, depending upon the particular skin disorder and the severity thereof. It is well within the purview of the skilled artisan, such as the dermatologist or other health care provider, to regulate dosages according to the patient needs. It is suggested as an example that the topical application range from about once per week to about four or five times daily, preferably from about three times per week to about three times daily, most preferably about once or twice per day. The composition for topical application will comprise from about 0.01% to about 5.0%, preferably from about 0.1% to about 2%, most preferably from about 0.1% to about 2% of the active compound or mixture of the compounds. By "chronic" application, it is meant herein that the period of topical application may be over the lifetime of the patient, preferably for a period of at least about one month, more preferably from about three months to about twenty years, more preferably from about six months to about ten years, more preferably still from about one year to about five years, thereby resulting in regulation of the anti-inflammatory response.

### 5. EXAMPLES

### EXAMPLE NO. 1

The following in vitro assay illustrates the effectiveness of the sialyl oligosaccharide derivative, 3' sialyl lactose, in inhibiting the adhesion of cells that express SLeX on their surfaces to cells expressing a selectin receptor.

The ability of 3'-sialyl lactose to inhibit cell adhesion was tested using an in vitro assay system. Neutrophils were activated with either interleukin-1 or phorbol ester, TPA, to induce expression of selections and binding to endothelial cells. The activated neutrophils were treated with either 3'-sialyl lactose at 0.01 and 0.02%, sucrose at 4% or a positive control, and then assayed for their ability to bind to PMN-endothelial cells.

**TABLE 1**

| Test Compound | | % Inhibition of Adhesion |
|---|---|---|
| 3'-Sialyl lactose | 0.01% | 39% |
| 3'-Sialyl lactose | 0.02% | 61% |
| Sucrose | 4.0% | 40% |
| Positive Control | | 100% |

Treating activated neutrophils with 0.02% 3' sialyl lactose effectively inhibited the ability of IL-1 induced neutrophils to bind PMN-endothelial cells. Therefore, in an *in vitro* assay 0.02% 3'-sialyl lactose is very effective in inhibiting the adhesion of cells that express SLeX on their surfaces to cells expressing a selectin receptor.

### EXAMPLE NO. 2

The following *in vivo* assay illustrates the effectiveness of sialyl oligosaccharide derivatives, 3'- and 6'-sialyl lactose, in preventing skin irritation. The in vivo assays used to assess efficacy of topical anti-irritants are known to those skilled in the art, examples of such assays may be found in Muizzuddin et al., 1990, J. Soc. Cosmet. Chem. 41:369-378, and Lubach et al. *in* Topical Corticoids, Maibach & Surber (eds.) Basel, Karger, 1992, pp. 26-41, both of which are incorporated herein by reference in their entirety. The skin anti-irritancy effects of 3'- and 6'- sialyl lactose were tested on 7 volunteers with a history of skin sensitivity to Balsam of Peru. The test compounds were applied to the ventral forearms of the panelists. The material was allowed to absorb for twenty minutes and then Balsam of Peru, an irritant, was applied to the test sites. Skin irritation was measured in terms of increase in skin redness.

The degree of redness was measured with the Minolta chromameter and compared with the positive and negative controls. The positive control was the color of skin treated with Balsam of Peru alone and the negative control was the skin treated with cola material challenged like the test material.

**TABLE 2**

| Test Compound | | % Decrease In Redness |
|---|---|---|
| 3'-Sialyl lactose | 0.1% | 69% |
| 3'-Sialyl lactose | 0.1% | 67% |
| 6'-Sialyl lactose | 0.1% | 57% |
| Sialic Acid | 0.1% | 31% |
| Lactose | 0.1% | 45% |

| CONTROLS | | |
|---|---|---|
| Cola | 10.0% | 65% |
| Hydrocortisone | 0.5% | 66% |
| Sialyl Lewis X | 0.1% | 66% |
| Balsam of Peru | | 0% |

Topical use of solutions containing 3'-sialyl lactose (3'-N acetylneuraminyl-lactose) showed significant activity in preventing irritation caused by a topical irritant. At one- hundredth the concentration of cola, 3'-sialyl lactose was more effective in preventing skin irritation, illustrating the extra ordinary beneficial effects of using sialyl oligosaccharide derivatives in topical compositions. The structural analog 6'-sialyl lactose at 0.1% was not as active as 3'-sialyl lactose, however showed significant activity in preventing skin irritation when compared to 10% cola. The building blocks of 3'-sialyl lactose; namely sialic acid and lactose, when tested alone, were ineffective at preventing irritation.

### EXAMPLE NO. 3

The following *in vivo* assay illustrates the effectiveness of 3'-sialyl oligosaccharide derivatives in preventing skin irritation. The skin anti-irritancy effects of various derivatives of 3'-sialyl lactose were tested on 7 volunteers with a history of skin sensitivity to Balsam of Peru. The test compounds benzyl sialyl lactose and ethyl sialyl lactose were applied to the ventral forearms of the panelists. The material was allowed to absorb for twenty minutes and then Balsam of Peru, an irritant, was applied on the test sites. Skin irritation was measured in terms of increase in skin redness.

The degree of redness was measured with the Minolta chromameter and compared with the positive and negative controls. The positive control was the color of skin treated with Balsam of Peru alone and the negative control was the skin treated with cola material challenged like the test material.

**TABLE 4**

| Test Compound | | % Decrease In Redness |
|---|---|---|
| Benzyl Sialyl lactose | 0.1% | 42.47% |
| Ethyl Sialyl lactose | 0.1% | 40.6% |

| CONTROLS | | |
|---|---|---|
| Cola | 10.0% | 68.7% |
| Balsam of Peru | | 0% |

At one-hundredth the concentration of cola, the 3'-sialyl lactose derivatives, lactose ethyl and benzyl sialyl lactose, demonstrated significant activity in reducing the development of skin irritation due to chemical irritant, exhibiting 40.6% and 42.47% activity respectively. Thus, demonstrating the beneficial effects of using these compounds in compositions suitable for topical application for the treatment of skin irritation.

The present invention is not to be limited in scope by the specific embodiments disclosed in the examples which are intended as illustrations of a few aspects of the invention and any embodiments which are functionally equivalent are with the scope of this invention. Indeed, various modifications of the invention in addition to those shown and described herein will become apparent to those skilled in the art and are intended to fall within the appended claims.

## Claims

1. Use of a sialyl lactose in the manufacture of a medicament for the treatment, by topical administration, of skin irritation, inflammation or inflammatory response in a mammal.

2. The use according to claim 1, wherein the skin irritation or inflammation is associated with a skin condition selected from the group consisting of atopic dermatitis, chronic urticaria lesions, inflammatory dermatoses, facial edema, Well's syndrome, psoriasis, eczema, pruritis, hyperkeratotic skin, dry skin, wrinkles, blemished skin, age-related skin changes, and acne.

3. The use according to claim 1, wherein the sialyl lactose is substantially purified.

4. The use according to claim 1, wherein said inflammatory response is skin reddening.

5. The use according to claim 1, wherein said sialyl lactose is selected from the group consisting of:
3' -sialyl lactose; and
6' -sialyl lactose;
or a cosmetically acceptable salt thereof.

6. The use according to claim 5 wherein said sialyl lactose is present in an amount of about 0.01 to about 5.0 wt % of the medicament.

7. The use according to claim 5 wherein said sialyl lactose is present in an amount of about 0.1 to about 2 wt % of the medicament.

## Patentansprüche

1. Verwendung einer Sialyllactose bei der Herstellung eines Medikaments für die Behandlung von Hautreizung, Entzündung oder entzündlicher Reaktion bei einem Säuger durch topische Verabreichung.

2. Verwendung nach Anspruch 1, wobei die Hautreizung oder Entzündung mit einem Hautleiden, ausgewählt aus der Gruppe, bestehend aus atopischer Dermatitis, Läsionen durch chronische Urticaria, entzündlichen Dermatosen, Gesichtsödem, Well-Syndrom, Psoriasis, Ekzem, Pruritus, hyperkeratotischer Haut, trockener Haut, Falten, unreiner Haut, altersabhängigen Hautveränderungen und Akne, verbunden ist.

3. Verwendung nach Anspruch 1, wobei die Sialyllactose im wesentlichen gereinigt ist.

4. Verwendung nach Anspruch 1, wobei die entzündliche Reaktion Hautrötung ist.

5. Verwendung nach Anspruch 1, wobei die Sialyllactose aus der Gruppe, bestehend aus:
3'-Sialyllactose und
6'-Sialyllactose;
oder einem kosmetisch verträglichen Salz davon ausgewählt ist.

6. Verwendung nach Anspruch 5, wobei die Sialyllactose in einem Anteil von etwa 0,01 bis etwa 5,0 Gew.-% des Medikaments vorhanden ist.

7. Verwendung nach Anspruch 5, wobei die Sialyllactose in einem Anteil von etwa 0,1 bis etwa 2 Gew.-% des Medikaments vorhanden ist.

## Revendications

1. Utilisation d'un lactose sialyl dans la fabrication d'un médicament pour le traitement, par administration topique, d'une irritation de la peau, d'une inflammation ou d'une réponse inflammatoire chez un mammifère.

2. Utilisation selon la revendication 1, dans laquelle l'irritation ou l'inflammation de la peau est associée avec une affection cutanée choisie dans le groupe constitué par une dermatite atopique, des lésions dues à un urticaire chronique, des dermatoses inflammatoires, un oedème facial, un syndrome de Well, un psoriasis, un eczéma, un prurit, une peau hyperkératosique, une peau sèche, des rides, une peau tachée, des changements cutanés liés à l'âge, et un acné.

3. Utilisation selon la revendication 1, dans laquelle le lactose sialyl est substantiellement purifié.

4. Utilisation selon la revendication 1, dans laquelle ladite réponse inflammatoire est une rubéfaction cutanée.

5. Utilisation selon la revendication 1, dans laquelle ledit lactose sialyl est choisi dans le groupe constitué par:
le 3'-lactose sialyl; et
le 6'-lactose sialyl;
ou un sel cosmétiquement acceptable de ceux-ci.

6. Utilisation selon la revendication 5, dans laquelle ledit lactose sialyl est présent en une quantité d'environ 0,01 à environ 5,0 % en poids du médicament.

7. Utilisation selon la revendication 5, dans laquelle ledit lactose sialyl est présent en une quantité d'environ 0,1 à environ 2 % en poids du médicament.
